# FASCICULE DE BREVET EUROPEEN

(11) **EP 1 079 732 B1**
(45) Date de publication et mention de la délivrance du brevet: **12.11.2003**
(21) Numéro de dépôt: 99920877.0
(22) Date de dépôt: 17.05.1999
(51) Int. Cl.: A61B 5/0215, A61B 5/00

(54) **SYSTEME DE MESURE DE PARAMETRES PHYSIQUES PAR UNE SONDE MEDICALE**
SYSTEM ZUR MESSUNG PHYSIKALISCHER PARAMETER MIT EINER MEDIZINISCHEN SONDE
SYSTEM FOR MEASURING PHYSICAL PARAMETERS WITH A MEDICAL PROBE

(30) Priorité: 18.05.1998 FR 9806235
(43) Date de publication de la demande: 07.03.2001
(73) Titulaire: COMMISSARIAT A L'ENERGIE ATOMIQUE, 75752 Paris Cédex 15 (FR); Absys, 92147 Clamart (FR); Tronic's Microsystems, 38054 Grenoble (FR)
(72) Inventeur: CLERC, Jean-Frédéric, F-38120 Le Fontanil (FR); PERRUCHOT, François, F-75015 Paris (FR); RENARD, Stéphane, F-38560 Champ-sur-Drac (FR)
(74) Mandataire: Ilgart, Jean-Christophe
(86) Numéro de dépôt international: FR9901168
(87) Numéro de publication internationale: WO99059467

(56) Documents cités:
- WO-A-93/09837
- WO-A-94/25105
- US-A- 4 127 110
- US-A- 4 519 401
- US-A- 4 846 191

## Description

### Domaine technique

La présente invention concerne la mesure d'un ou de plusieurs paramètres physiques par une sonde médicale.

### Etat de la technique antérieure

Les sondes ou cathéters médicaux ont pour fonctions de délivrer un fluide à l'intérieur du corps d'un patient et/ou d'y mesurer certains paramètres physiques. Ces paramètres peuvent être des paramètres liés à la délivrance du fluide comme le débit ou la composition chimique ou simplement des paramètres d'environnement, tel que pression, température, humidité ou pH. Ce fluide peut être un liquide comme par exemple pour des mesures intravasculaires ou urinaires aussi bien qu'un gaz comme pour les mesures pulmonaires.

Pour la mesure de la pression distale, soit en bout de sonde (à l'intérieur du corps), la méthode couramment utilisée consiste à déporter la mesure en proximal soit à l'extérieur de la sonde afin de mesurer la pression à l'aide d'un capteur électronique classique n'ayant pas besoin d'être miniaturisé. Deux principales techniques peuvent être utilisées pour l'interface permettant de transmettre la pression : par mandrin liquide ou par mandrin gazeux. Cependant, ces techniques posent des problèmes en terme de fiabilité, de facilité d'utilisation et de précision. L'utilisation directe du gaz comme interface entraine un amortissement du signal mesuré et manque de fiabilité car il existe un risque de bouchage de la prise de pression par les sécrétions du patient. La technique du mandrin liquide a l'avantage de reposer sur l'utilisation d'une interface incompressible. Elle est utilisable pour des mesures sur des liquides mais aussi pour des mesures de pression gazeuse à l'aide d'une interface adéquate comme proposé par le brevet US-A-4 813 431. Il existe cependant un risque d'apparition de bulles d'air dans la colonne qui est un danger potentiel pour le patient. Il faut donc procéder le cas échéant à des opérations de débullage. De plus, la colonne liquide entraîne, à cause de la densité élevée de l'eau, l'apparition d'un décalage entre la pression lue et la pression interne qui dépend de la différence d'altitude entre les deux points. Le document WO 95/22 280 a proposé une méthode par mesure laser pour estimer cette différence, mais cette technique demande un appareillage peu pratique et coûteux à utiliser.

Les sondes ou cathéters sont utilisés pour différentes applications dans le domaine médical, mais les besoins et les problèmes techniques sont globalement identiques. Un exemple particulièrement significatif est le cas de la ventilation artificielle.

La ventilation artificielle d'un patient en réanimation peut se prolonger sur plusieurs semaines, voire plusieurs mois. Le réanimateur utilise souvent les modes ventilatoires "en pression" dans lesquels le ventilateur doit atteindre un niveau de pression (pression contrôlée) ou faciliter l'inspiration du patient (pression assistée). Le respirateur est guidé par les réactions du patient qui lui sont transmises par des capteurs de débit ou de pression. Il est souhaitable de limiter l'agressivité des modes ventilatoires afin de ne pas aggraver l'état du patient, puis d'accélérer sa guérison et de favoriser ainsi le retour progressif à l'autonomie respiratoire. Lorsque le sujet dispose encore, ou de nouveau, de ses réflexes respiratoires, la délivrance du gaz par le respirateur est déclenché par le patient. La sensibilité et la fiabilité des capteurs et en particulier des capteurs de pression sont donc essentielles pour une bonne adaptation de la ventilation artificielle aux besoins réels du patient.

Actuellement, les capteurs de pression sont situés sur le circuit externe qui relie le patient au respirateur artificiel. Les signaux fournis par ces capteurs ne reflètent pas les conditions réelles in vivo à cause du décalage dû aux éléments de raccordement, en particulier la sonde d'intubation. Ce phénomène est particulièrement important lors de la phase de sevrage du patient qui correspond à des débits instantanés élevés. La technique du mandrin gazeux est proposée commercialement par tous les fabricants de sonde mais n'est pas utilisée pour l'asservissement direct du respirateur à cause de son manque de fiabilité.

La mesure directe et fiable de la pression à l'intérieur des voies respiratoires constitue un progrès significatif pour la sécurité du patient et ouvre la voie vers la mise au point de modes ventilatoires plus performants qui permettront de réduire la durée moyenne de séjour en services de soins intensifs et auront donc un impact positif sur les coûts hospitaliers. L'utilité de telles techniques est accrue avec les nouveaux modes de ventilation à fréquence élevée, pour l'adulte mais surtout pour le petit enfant pour lesquels ils n'existe pas aujourd'hui de moyen de surveillance précis utilisable en routine.

Un autre exemple concerne l'urodynamique. Certains examens d'urologie demandent l'injection d'un liquide dans la vessie du patient avec le suivi de l'évolution de la pression. Le coût actuel des cathéters instrumentés intégrant des capteurs de pression électronique, qui sont de plus très fragiles, est élevé. Ils sont donc réservés essentiellement à des applications de recherche. Aussi la mesure de la pression au niveau de l'alimentation en liquide physiologique est-elle préférée. Mais, à cause des problèmes de débullage et de décalage de pression provenant de la hauteur de la colonne de liquide injecté, cette technique est difficile à mettre en oeuvre ce qui la rend peu fiable.

Un autre exemple concerne des sondes utilisées dans le domaine cardiovasculaire. La pression artérielle est mesurée à l'extérieur à l'extrémité proximale d'un cathéter rempli de liquide physiologique sous pression. Cette technique nécessite l'utilisation d'une poche de sérum pressurisée et d'une vanne de régulation qui encombrent l'environnement du patient. Cette application est l'illustration de l'utilisation d'un cathéter contenant un capteur de pression, mais sans la délivrance d'un fluide.

Il existe donc une demande, particulièrement en mesure de pression, pour une technique permettant d'intégrer directement le capteur électronique au bout de la sonde ou du cathéter à l'endroit même où la mesure est nécessaire. Ce besoin s'étend à une mesure de plusieurs paramètres en bout de cathéter.

Pour des raisons d'hygiène, les sondes sont de préférence à usage unique. On cherche donc à les fabriquer à un prix de revient modéré. Elles sont fabriquées par des techniques d'extrusion qui permettent d'atteindre les coûts objectifs même pour des productions en moyenne série. Ces techniques sont contraignantes car elles imposent une symétrie de la sonde par rapport à l'axe longitudinal en terme de matériaux et formes. Des techniques permettant de fabriquer une sonde avec une extrémité dans un matériau différent de celui du corps de la sonde sont connues (voir par exemple les documents US-A-3 890 976 et WO 94/00174). Les techniques de mesure de la pression au bout d'une sonde à l'aide d'un capteur électronique implanté directement dans la sonde et relié par des câbles à l'extérieur sont connues. Elles permettent de mesurer la pression intratrachéale (cf. WO 94/22518) ou la pression artérielle (cf. WO 97/17888). Des techniques de multiplexage ont été proposées pour permettre l'utilisation d'une seule paire de câbles (voir le brevet US-A-4 432 372). Cependant, ces techniques entraînent un surcoût important à cause de la fixation du capteur dans la sonde et des connexions électriques qui ne sont pas directement compatibles avec les techniques d'extrusion. Leur utilisation est donc limitée à quelques applications.

De manière générale, et en particulier pour la pression, la fabrication des microcapteurs repose sur les microtechnologies qui sont en plein développement grâce aux progrès de l'industrie de la micro-électronique. Les techniques développées aujourd'hui permettent d'intégrer sur des composants électroniques des fonctions mécaniques et de concevoir des capteurs électroniques miniatures. Ces capteurs ont l'avantage, par rapport aux capteurs mécaniques traditionnels, d'être plus sensibles, plus fiables et plus polyvalents puisque susceptibles d'être associés directement à une unité de traitement du signal.

Dans le cas particulier des capteurs de pression, différentes techniques de mesure de la déformation d'une membrane ont été proposées pour ces systèmes. Les techniques piézorésistives permettent de mesurer les déformations d'un élément piézorésistif déposé à la surface de la membrane. La détermination de la pression se fait par mesure de variation de résistance. Cette technique est aujourd'hui limitée par la taille minimum du capteur de pression qu'elle impose et la consommation du système de détection. La mesure de déformation par système optique (voir le brevet US-A-5 546 935) permet de simplifier les problèmes de connexions électriques mais est difficilement utilisable pour la mesure de plusieurs paramètres et ne permet pas d'intégrer un traitement in situ du signal. Les techniques capacitives sont aujourd'hui les plus prometteuses en terme d'encombrement et de consommation électrique. Elles demandent cependant l'ajout, à proximité immédiate, d'une électronique de traitement de la variation de capacité.

Ces microcapteurs sont classiquement connectés par des fils de liaison ou par une fibre optique à leurs dispositifs de traitement de données. Le brevet US-A-4 127 110 décrit une variante de microcapteur sans fil de liaison qui est utilisée dans le cas de capteurs capacitifs pour la mesure de la pression intracrânienne. La capacité du capteur est utilisée comme base d'un circuit LC dont la fréquence de résonance permet de mesurer les variations de pression. Cette technique est cependant limitée à l'acquisition d'un seul paramètre et la transmission de la mesure est nécessairement analogique, ce qui limite la précision. Son extension à la mesure de plusieurs paramètres a été proposée dans le brevet US-A-4 556 063 dans le cas de circuits implantés possédant une alimentation électrique par pile. Cette technique est aujourd'hui utilisée pour la programmation des stimulateurs cardiaques. Suite au progrès de la miniaturisation, il est possible d'appliquer des techniques de téléalimentation et de télétransmission à l'aide de circuits miniatures. Ces techniques présentent l'avantage d'apporter une plus grande souplesse en terme de traitement. L'application pour des systèmes ne comportant que des circuits de mesure passifs est connue pour des systèmes implantables (voir pour exemple le brevet US-A-5 704 352).

Le principal problème posé pour l'utilisation des composants à base de microsystèmes dans les sondes médicales est le coût de leur intégration

### Exposé de l'invention

Afin de remédier à ce problème, il est proposé de réaliser la partie mécanique de la sonde (la tige) selon une technique conventionnelle et bon marché, sans incorporation de composant électronique ou de connecteur électrique, et de lui adjoindre un module de mesure électronique comportant un ou plusieurs capteurs, une électronique rapprochée miniaturisée permettant le traitement du signal de mesure et un composant assurant la transmission des valeurs mesurées et la réception d'un signal de téléalimentation.

L'invention a donc pour objet un système de mesure d'au moins un paramètre physique dans un endroit du corps d'un patient accessible par une sonde médicale, comprenant une sonde médicale équipée distalement d'un capteur dudit paramètre et des moyens pour délivrer un signal électrique représentatif dudit paramètre, et recueilli par le capteur, à un dispositif de traitement de données externe au patient, caractérisé en ce que :
- ladite sonde est constituée par une tige comportant des moyens de fixation d'un module de mesure électronique,
- le capteur dudit paramètre est inclus dans le module de mesure électronique qui inclut également d'autres éléments constitués par des moyens électroniques associés au capteur pour fournir un signal de mesure, des moyens de télétransmission du signal de mesure, des moyens d'alimentation électrique des moyens électroniques associés au capteur et des moyens de télétransmission, le module de mesure comportant en outre des moyens de fixation complémentaires de ceux de la tige de la sonde,
- les moyens pour délivrer un signal électrique représentatif dudit paramètre au dispositif de traitement de données sont des moyens récepteurs disposés de manière à pouvoir recevoir le signal de mesure transmis par les moyens de télétransmission,
- les moyens de télétransmission comportent une antenne disposée sous forme d'enroulement,
- les moyens d'alimentation électrique comportent un circuit pouvant être chargé par téléalimentation grâce audit enroulement.

Les moyens de fixation de la tige peuvent comprendre un logement permettant d'insérer le module de mesure. Si la tige est creuse, ledit logement peut être prévu dans la paroi interne de la tige. Le module de mesure peut être en forme d'anneau, ledit logement constituant une gorge annulaire dans la tige. Si le module de mesure est un anneau fermé, la tige peut être réalisée en un matériau suffisamment élastique pour que le module de mesure puisse être introduit dans son logement par déformation de la tige. La tige peut aussi comporter une restriction contre laquelle le module de mesure vient en butée. Dans ce cas, la tige peut également comprendre un système d'ancrage retenant le module de mesure dans son logement. Le module de mesure peut être un anneau ouvert se mettant en place dans ledit logement à la manière d'un clips.

Les moyens de fixation entre le module de mesure et la tige peuvent comprendre des surfaces de mise en contact entre le module de mesure et la tige. Les moyens de fixation entre le module de mesure et la tige peuvent comprendre une substance adhésive ou reposer sur des techniques de surmoulage.

Les moyens récepteurs peuvent être disposés sur l'extrémité de la sonde qui est externe au patient. Ils peuvent aussi être des moyens susceptibles d'être disposés sur le corps du patient.

Les moyens de télétransmission et les moyens de réception peuvent être des moyens de communication par radiofréquence, des moyens de communication par infrarouge ou des moyens de communication ultrasonores.

Avantageusement, le module de mesure comporte un plan de connexion pourvu de pistes conductrices permettant d'assurer les liaisons électriques entre les différents éléments du module de mesure. Ce plan de connexion peut être constitué par un support flexible enroulé en forme de tube et noyé dans une substance de moulage. Les éléments du module de mesure peuvent avoir des contacts électriques soudés sur des pistes conductrices appropriées parmi lesdites pistes conductrices. Ils peuvent être des éléments disposés dans le module de mesure par insertion, ladite insertion produisant des contacts électriques entre ces éléments et des pistes conductrices appropriées parmi lesdites pistes conductrices.

Les moyens de télétransmission et les moyens de réception étant des moyens de communication par radiofréquence, le module de mesure peut comprendre une antenne réalisée par une métallisation déposée sur le support flexible. L'antenne peut être un élément rapporté, connecté au plan de connexion et noyé dans la substance de moulage. Elle peut encore être disposée autour du module de mesure.

La sonde médicale peut être une sonde d'intubation apte à délivrer un gaz dans le cas d'une ventilation artificielle. Elle peut aussi être une sonde urinaire apte à servir à des mesures urodynamiques. Elle peut encore être un cathéter apte à mesurer la pression intravasculaire.

### Brève description des dessins

L'invention sera mieux comprise et d'autres avantages et particularités apparaîtront à la lecture de la description qui va suivre, donnée à titre d'exemple non limitatif, accompagnée des figures annexées parmi lesquelles :
- la figure 1 illustre la manière dont une sonde médicale du système de mesure selon la présente invention peut recevoir le module de mesure électronique qui lui est associé ;
- la figure 2 montre une sonde médicale du système de mesure, selon la présente invention, équipée de son module de mesure électronique ;
- les figures 2A et 3 illustrent d'autres manières d'associer une sonde médicale et un module de mesure électronique pour un système de mesure selon la présente invention ;
- la figure 4 est un schéma fonctionnel du module de mesure électronique du système de mesure selon la présente invention ;
- la figure 5 est une vue en coupe d'un module de mesure électronique pour un système de mesure selon la présente invention ;
- la figure 6 est illustrative de la manière de réaliser un module de mesure électronique pour un système de mesure selon la présente invention ;
- les figures 7 et 8 montrent deux variantes de module de mesure électronique pour système de mesure selon l'invention, équipées d'antennes extérieures ;
- la figure 9 représente un module de mesure électronique pour un système de mesure selon l'invention, ce module étant conçu pour une sonde médicale à paroi de faible épaisseur.

### Description détaillée de modes de réalisation de l'invention

Le système de mesure selon la présente invention présente l'avantage d'utiliser une sonde médicale simple à fabriquer et peu onéreuse. On la réalise avantageusement par extrusion selon des techniques bien connues de l'homme de l'art.

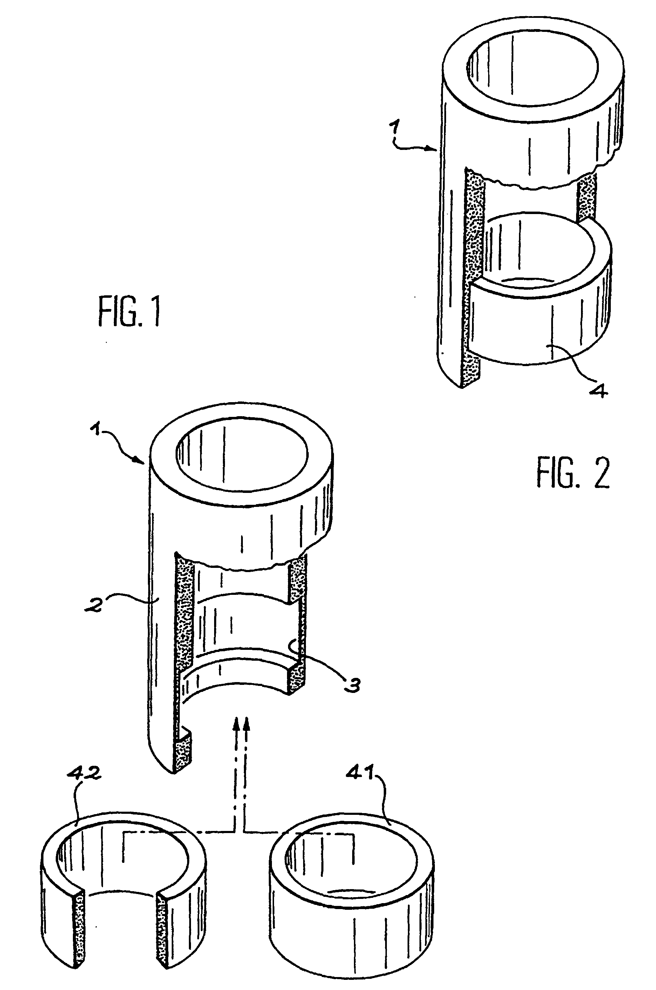

Comme le montre la figure 1, la sonde 1 peut être constituée par un tube 2 pourvu, près de l'une de ses extrémités, d'un logement permettant l'insertion d'un module de mesure électronique. Dans l'exemple représenté, ce logement 3 constitue une gorge annulaire prévue dans la paroi interne du tube 2. Le logement 3 est apte à recevoir un module de mesure de forme adaptée à ce logement. Le module de mesure peut avantageusement se présenter sous la forme d'un anneau pour préserver une communication de fluide entre les deux extrémités de la sonde.

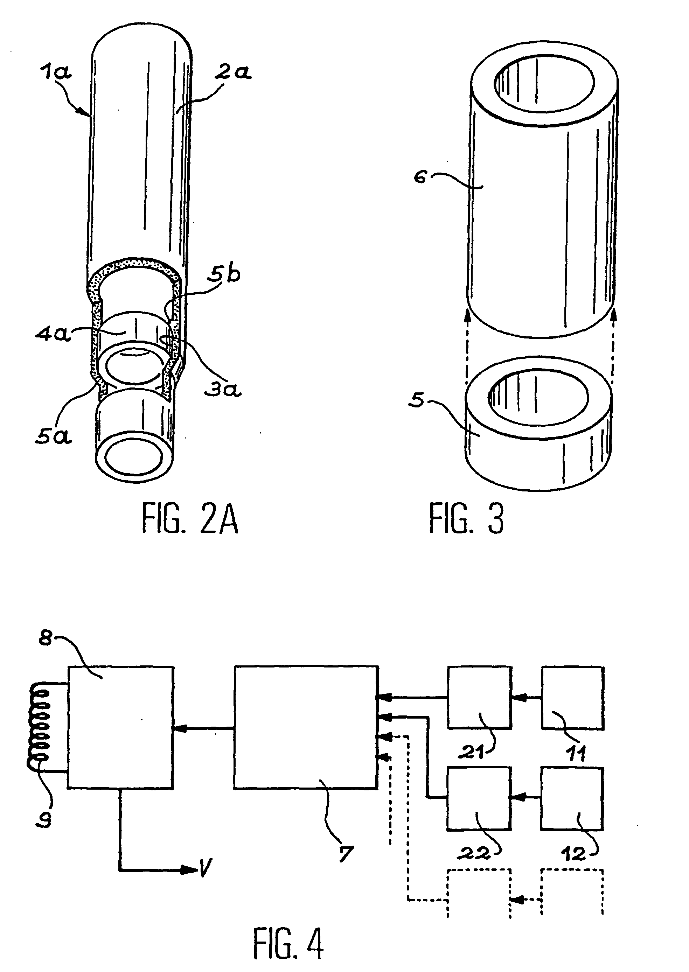

La figure 2 montre une sonde médicale 1 équipée de son module de mesure 4. Comme le montre la figure 1, le module de mesure peut être un anneau fermé 41 ou un anneau ouvert 42. Si le module de mesure est un anneau fermé, la tige 1 est conçue de manière à pouvoir recevoir le module par déformation élastique de sa paroi. Si le module de mesure est un anneau ouvert, celui-ci peut être inséré dans son logement en réduisant par pression son diamètre à la manière d'un clips. Une autre façon d'insérer le module de mesure dans la tige de la sonde est montrée à la figure 2A. Le tube 2a de la sonde la présente une restriction 5a à son extrémité distale. Le module de mesure 4a, de diamètres interne et externe identiques à ceux de la restriction, est inséré dans le tube 2a par son extrémité proximale. Il est poussé jusqu'à venir en butée contre la restriction 5a. Un système d'ancrage 5b permet de maintenir le module en place, dans son logement 3a, pour éviter sa remontée.

Dans ces modes de réalisation, l'instrumentation de la sonde peut se faire de manière stérile avant son utilisation, le médecin pouvant choisir un module adapté au besoin de l'examen qu'il pratique.

La figure 3 représente un autre mode de fixation d'un module de mesure sur la tige d'une sonde médicale. Selon cette variante, le module de mesure 5, de forme tubulaire, est fixé à l'extrémité de la tige 6 constituant la sonde, également de forme tubulaire. La fixation peut se faire au moyen d'une substance adhésive ou par une technique de surmoulage. Les pièces 5 et 6 peuvent être simplement mises bout à bout. Il peut également y avoir pénétration partielle ou totale du module de mesure 5 dans le tube 6. La fixation peut alors être réalisée lors d'une étape de fabrication du système de mesure.

Le module de mesure peut comprendre plusieurs capteurs pour la mesure d'autant de paramètres physiques et des composants électroniques associés à ces capteurs, ainsi que les liaisons électriques entre ces différents éléments. Dans le cas d'une transmission des mesures par radio-fréquence, il comporte également une antenne d'émission. La transmission des mesures peut se faire grâce à une antenne de réception. L'antenne de réception peut être externe au patient, la transmission se faisant alors à travers la peau du patient. Le récepteur peut être fixé sur la peau du patient comme une électrode d'électrocardiographie. L'antenne de réception peut aussi être placée dans la paroi de la sonde ou à l'intérieur de la sonde. Le récepteur, qu'il soit externe au patient (fixé sur sa peau) ou interne au patient (dans la sonde), est relié par des fils de liaison à un dispositif de traitement des données fournies par les capteurs. Cette liaison peut être faite par l'intermédiaire d'un dispositif d'analyse des signaux ou directement à la machine de monitorage. Le récepteur, le dispositif d'analyse ou la machine de monitorage peuvent comprendre des capteurs de référence, en particulier pour la pression.

La figure 4 présente un schéma fonctionnel possible pour la partie électronique du capteur de mesure. Chaque capteur 11, 12, ... est associé à un circuit de conversion numérique respectivement 21, 22, ... dont les sorties sont connectées aux entrées d'un circuit de traitement du signal 7. Différents paramètres physiques peuvent ainsi être mesurés : la pression, la température, la composition chimique, le pH, le taux d'humidité pour un gaz. Le circuit de traitement du signal 7 délivre un signal représentatif des différentes mesures effectuées à un circuit d'émission 8. Les mesures peuvent être transmises séparément ou de manière combinée sous forme d'une variable multiparamètre. L'émission s'effectue grâce à l'antenne 9.

L'alimentation électrique des composants électroniques du module de mesure est assurée par télétransmission. En effet, si l'antenne 9 est disposée sous forme d'enroulement, il est possible d'alimenter le module de mesure par induction. Dans ce cas, au circuit d'émission 8 on peut ajouter un circuit de conversion d'une tension alternative en une tension continue d'alimentation V.

Un système d'autocalibration peut être intégré dans le module de mesure pour prendre en compte les décalages introduits dans le système de conversion. Un signal d'authentification du module peut aussi être prévu dans la transmission des signaux de mesure.

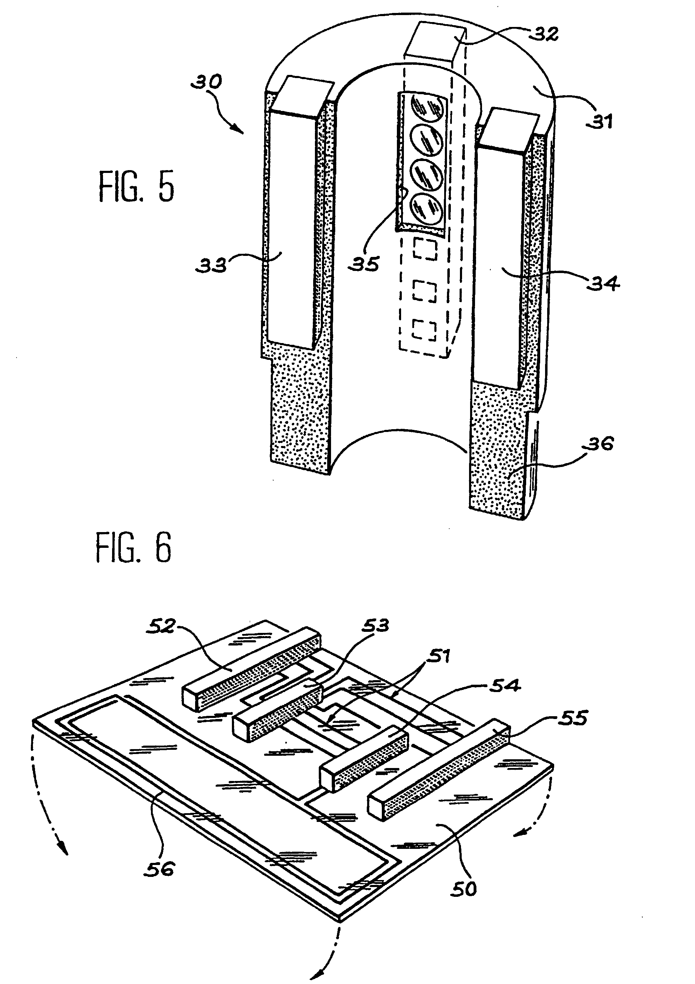

La figure 5 montre, en coupe longitudinale, un module de mesure 30 selon la présente invention. Le corps annulaire 31 du module sert de support mécanique à l'ensemble des composants électroniques constituant sa partie électronique. Il est obtenu par moulage. Des emplacements sont prévus dans la masse du corps annulaire 31 pour loger un composant 32 contenant les différents capteurs et des composants 33 et 34 regroupant les autres circuits. Les différents composants et les pistes pour les liaisons électriques sont répartis sur un plan de connexion. Les capteurs ne requérant pas de contact direct avec le milieu à caractériser (capteur de température par exemple) sont noyés dans le corps annulaire 31. Pour les autres, une fenêtre 35 est prévue dans le corps annulaire 31. Une protection de surface est néanmoins effectuée pour éviter les problèmes d'humidité sur les contacts électriques et l'influence d'un dépôt éventuel sur la surface sensible des capteurs non noyés.

Pour la mesure de pression, les capteurs sont de préférence des micro-capteurs de pression absolue, capacitifs, optimisés pour fonctionner dans la gamme 700-1400 mbar. Ils sont pourvus de deux ou trois contacts donnant accès à une capacité de mesure et éventuellement à une capacité de référence. Une électronique de traitement particulière est associée à chaque type de capteur en fonction du type de paramètre électrique associé à chaque capteur : mesure de résistance, de capacité ou d'inductance. Le traitement comprend au minimum une conversion numérique du signal mesuré.

Les capteurs capacitifs sont associés à des circuits ASIC (circuits Intégrés pour Applications Spécifiques) qui permettent de comparer une capacité variable à une capacité de référence. La différence entre les deux capacités est directement traduite en un signal numérique en utilisant par exemple un système basé sur le principe des capacités commutées. Le même principe peut être appliqué à des mesures de résistance.

Le module de mesure 30 comporte une partie 36 de diamètre extérieur réduite permettant de placer le bobinage de l'antenne si nécessaire.

La figure 6 montre une étape de la réalisation d'un module de mesure pour un système de mesure selon l'invention. Le plan de connexion est constitué par un support flexible 50, du type Kapton® , supportant sur l'une de ses faces des pistes conductrices 51 permettant de relier électriquement les composants électroniques du module. Ces pistes peuvent être réalisées par métallisation, par sérigraphie ou en cuivre électrodéposé. Les composants électroniques, par exemple les composants 52, 53, 54 et 55 sont fixés sur le support flexible 50. Pour les capteurs demandant un contact avec le fluide dont on veut mesurer des paramètres physiques, une fenêtre est prévue dans le support flexible.

L'antenne 56 peut également être déposée sur le support flexible 50 par les mêmes techniques que pour les pistes.

Dans une première étape, les composants 52 à 55 sont posés sur le support flexible 50, les faces des composants comportant les contacts électriques et les zones sensibles des capteurs étant tournées vers le support flexible. Un léger chauffage permet de faire fondre des microbilles fusibles déposées sur chaque contact électrique lors de leur fabrication ou sur les pistes du support flexible.

Dans une seconde étape, le support flexible est courbé comme cela est suggéré par des flèches sur la figure 6 et est placé à l'intérieur d'un moule pour obtenir le corps du module. Cette opération permet de donner au module la rigidité souhaitée.

Les connexions mécaniques et électriques des composants peuvent aussi s'effectuer après mise en forme du support.

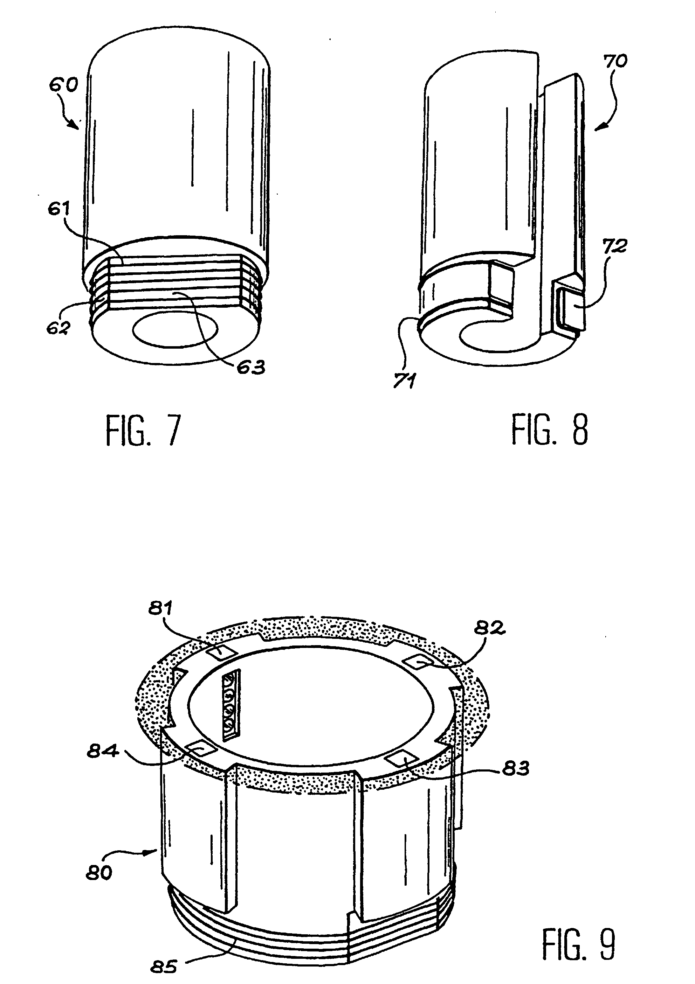

Si l'antenne d'émission n'est pas réalisée directement sur le support flexible, elle peut être insérée lors du moulage. L'antenne d'émission peut également être disposée autour du corps du module de mesure comme cela est illustré par les figures 7, 8 et 9. Dans tous les cas, la géométrie du corps du module est telle que la connexion de l'antenne au reste du circuit électronique du module se fait également dans le plan de connexion.

Dans un autre mode de réalisation, les composants électroniques sont pré-assemblés ou intégrés au sein d'un seul circuit. Ce circuit unique est alors intégré au corps du module et relié à l'antenne suivant les techniques décrites précédemment.

La figure 7 représente un module de mesure 60 de forme tubulaire et dont l'antenne d'émission 61 est enroulée à l'extérieur du corps du module. L'antenne 61 est enroulée sur une partie 62 de diamètre réduit du corps du module de mesure. Un méplat 63 est prévu sur la partie 62 pour permettre le branchement direct de l'antenne dans le plan de connexion.

La figure 8 représente un module de mesure 70 ouvert longitudinalement à la manière d'un clips et dont l'antenne 71 est enroulée autour d'une partie proéminente 72. L'axe de l'antenne peut être parallèle ou perpendiculaire à l'axe de la sonde.

Dans le cas d'une sonde dont la paroi est de faible épaisseur et si un module de mesure de forme annulaire doit être inséré à l'intérieur de cette sonde, il est préférable de donner au module la forme illustrée par la figure 9. Le module de mesure 80 représenté sur cette figure possède des régions d'épaisseurs différentes. Les régions contenant les composants électroniques 81, 82, 83 et 84 sont d'épaisseur adaptée à l'intégration de ces composants. Les régions intermédiaires à ces régions contenant des composants sont d'épaisseur plus faible. En grisé est figurée l'épaisseur de la sonde correspondant à ce module de mesure. Aux régions de faible épaisseur du module de mesure correspondent des régions épaisses de la sonde. Cette façon de procéder permet de ne pas fragiliser la sonde sur toute son épaisseur. Sur la figure 9, on reconnaît l'antenne 85 qui est enroulée autour du module 80.

Le module de mesure du système de mesure selon la présente invention peut aussi être une pièce moulée en plusieurs parties contenant des emplacements pour les capteurs et les autres composants électroniques.

Une protection des capteurs non noyés dans le corps du module de mesure et de l'antenne d'émission, si celle-ci est apparente, peut se faire par dépôt d'un gel et/ou par fixation d'un mince film d'élastomère biocompatible polymérisé in situ ou fixé par thermosoudage.

## Revendications

1. Système de mesure d'au moins un paramètre physique dans un endroit du corps d'un patient accessible par une sonde médicale, comprenant une sonde médicale (1, 6) équipée d'un capteur dudit paramètre et des moyens pour délivrer un signal électrique représentatif dudit paramètre, et recueilli par le capteur, à un dispositif de traitement de données externe au patient, dans lequel :
- ladite sonde (1, 6) est constituée par une tige (2) comportant des moyens de fixation fixant un module de mesure électronique (4,5,30,60,70,80) à la tige,
- le capteur (11, 12) dudit paramètre est inclus dans le module de mesure électronique (4,5,30,60,70,80) qui inclut également d'autres éléments constitués par des moyens électroniques (21, 22, 7) associés au capteur (11, 12) pour fournir un signal de mesure, des moyens de télétransmission (8, 9) du signal de mesure, des moyens d'alimentation électrique des moyens électroniques associés au capteur et des moyens de télétransmission,
**caractérisé en ce que :**
- le module de mesure comporte en outre des moyens de fixation complémentaires de ceux de la tige (2) de la sonde,
- les moyens pour délivrer un signal électrique représentatif dudit paramètre au dispositif de traitement de données sont des moyens récepteurs disposés de manière à pouvoir recevoir le signal de mesure transmis par les moyens de télétransmission (8, 9),
- les moyens de télétransmission comportent une antenne (9) disposée sous forme d'enroulement,
- les moyens d'alimentation électrique comportent un circuit dont la charge se fait par téléalimentation grâce audit enroulement,
- le capteur est situé en partie distale de la sonde médicale.

2. Système de mesure selon la revendication 1, **caractérisé en ce que** les moyens de fixation de la tige (2, 2a) comprennent un logement (3, 3a) permettant d'insérer le module de mesure (41, 42, 4a).

3. Système de mesure selon la revendication 2, **caractérisé en ce que** la tige (2, 2a) étant creuse, ledit logement (3, 3a) est prévu dans la paroi interne de la tige.

4. Système de mesure selon la revendication 3, **caractérisé en ce que** le module de mesure (41, 42) est en forme d'anneau, ledit logement (3) constituant une gorge annulaire dans la tige (2).

5. Système de mesure selon la revendication 4, **caractérisé en ce que** le module de mesure (41) étant un anneau fermé, la tige (2) est réalisée en un matériau suffisamment élastique pour que le module de mesure (41) puisse être introduit dans son logement (3) par déformation de la tige (2).

6. Système de mesure selon la revendication 4, **caractérisé en ce que** le module de mesure (42) est un anneau ouvert se mettant en place dans ledit logement (3) à la manière d'un clips.

7. Système de mesure selon la revendication 3, **caractérisé en ce que** la tige (2a) comporte une restriction (5a) contre laquelle le module de mesure (4a) vient en butée.

8. Système de mesure selon la revendication 7, **caractérisé en ce que** la tige (2a) comprend un système d'ancrage (5b) retenant le module de mesure (4a) dans son logement (3a).

9. Système de mesure selon la revendication 1, **caractérisé en ce que** les moyens de fixation entre le module de mesure (5) et la tige (6) comprennent des surfaces de mise en contact entre le module de mesure et la tige.

10. Système de mesure selon la revendication 9, **caractérisé en ce que** les moyens de fixation entre le module de mesure (5) et la tige (6) comprennent une substance adhésive.

11. Système de mesure selon l'une quelconque des revendications précédentes, **caractérisé en ce que** les moyens récepteurs sont disposés sur l'extrémité proximale de la sonde.

12. Système de mesure selon l'une quelconque des revendications 1 à 10, **caractérisé en ce que** les moyens récepteurs sont des moyens susceptibles d'être disposés sur le corps du patient.

13. Système de mesure selon l'une quelconque des revendications 1 à 12, **caractérisé en ce que** les moyens de télétransmission (8, 9) et les moyens de réception sont des moyens de communication par radiofréquence.

14. Système de mesure selon l'une quelconque des revendications 1 à 12, **caractérisé en ce que** les moyens de télétransmission et les moyens de réception sont des moyens de communication par infrarouge.

15. Système de mesure selon l'une quelconque des revendications 1 à 12, **caractérisé en ce que** les moyens de télétransmission et les moyens de réception sont des moyens de communication ultrasonores.

16. Système de mesure selon l'une quelconque des revendications précédentes, **caractérisé en ce que** le module de mesure comporte un plan de connexion pourvu de pistes conductrices (51) permettant d'assurer les liaisons électriques entre les différents éléments (52, 53, 54, 55) du module de mesure.

17. Système de mesure selon la revendication 16, **caractérisé en ce que** le plan de connexion est constitué par un support flexible (50) enroulé en forme de tube et noyé dans une substance de moulage.

18. Système de mesure selon la revendication 17, **caractérisé en ce que** les éléments (52, 53, 54, 55) du module de mesure ont des contacts électriques soudés sur des pistes conductrices (51) appropriées parmi lesdites pistes conductrices.

19. Système de mesure selon l'une des revendications 16 ou 17, **caractérisé en ce que** les éléments (32, 33, 34) du module de mesure sont des éléments disposés dans le module de mesure par insertion, ladite insertion produisant des contacts électriques entre ces éléments et des pistes conductrices appropriées parmi lesdites pistes conductrices.

20. Système de mesure selon la revendication 17, **caractérisé en ce que**, les moyens de télétransmission et les moyens de réception étant des moyens de communication par radiofréquence, le module de mesure comprend une antenne réalisée par une métallisation (56) déposée sur le support flexible (50).

21. Système de mesure selon l'une quelconque des revendications 16 à 18, **caractérisé en ce que**, les moyens de télétransmission et les moyens de réception étant des moyens de communication par radiofréquence, le module de mesure comprend ladite antenne qui est un élément rapporté, connecté au plan de connexion et noyé dans la substance de moulage.

22. Système de mesure selon l'une quelconque des revendications 16 à 19, **caractérisé en ce que**, les moyens de télétransmission et les moyens de réception étant des moyens de communication par radiofréquence, le module de mesure (60, 70, 80) comprend ladite antenne (61, 71, 85) disposée autour du module de mesure.

23. Système de mesure selon l'une quelconque des revendications précédentes, **caractérisé en ce que** ladite sonde est une sonde d'intubation apte à délivrer un gaz dans le cas d'une ventilation artificielle.

24. Système de mesure selon l'une quelconque des revendications 1 à 22, **caractérisé en ce que** ladite sonde est une sonde urinaire apte à servir à des mesures urodynamiques.

25. Système de mesure selon l'une quelconque des revendications 1 à 22, **caractérisé en ce que** ladite sonde est un cathéter apte à mesurer 1a pression artérielle.

## Claims

1. System for measuring at least one physical parameter in a place in a patient's body to which a medical probe has access, comprising a medical probe (1, 6) equipped with a sensor of said parameter and means for emitting an electrical signal that represents said parameter and that is received by the sensor, to a data processing device outside the patient's body, wherein:
- said probe (1, 6) consists of a rod (2) comprising means to fasten it to an electronic measurement unit (4, 5, 30, 60, 70, 80),
- the sensor (11, 12) of said parameter is included in the electronic measurement unit (4, 5, 30, 60, 70, 80) that also includes other parts consisting of electronic means (21, 22, 7) associated with the sensor (11, 12) to provide a measurement signal, means for remote transmission (8, 9) of the measurement signal, power supply means for the electronic means associated with the sensor and remote transmission means, **characterized in that**
- the measurement unit also comprising additional fastening means besides those of the rod (2) of the probe,
- the means for emitting an electronic signal representing said parameter to the data processing device are receiver means positioned such that they are capable of receiving the measurement signal emitted by the remote transmission means (8, 9),
- the remote transmission means comprise a coil-shaped antenna (9),
- the power supply means comprise a circuit capable of being charged by a remote power supply using said coil,
- the sensor being located in the distal part of the medical probe.

2. Measurement system according to claim 1, **characterized in that** the fastening means of the rod (2, 2a) comprise a housing (3, 3a) that enables the measurement unit (41, 42, 4a) to be inserted.

3. Measurement system according to claim 2, **characterized in that** as the rod (2, 2a) is hollow, said housing (3, 3a) is provided inside the inner wall of the rod.

4. Measurement system according to claim 3, **characterized in that** the measurement unit (41, 42) is ring shaped, said housing (3) constituting an annular throat in the rod (2).

5. Measurement system according to claim 4, **characterized in that** as the measurement unit (410 is a closed ring the rod (2) is produced in a material that is sufficiently elastic to enable the measurement unit (41) to be inserted into the housing (3) by distortion of the rod (2).

6. Measurement system according to claim 4, **characterized in that** the measurement unit (42) is an open ring that clips into place in said housing (3).

7. Measurement system according to claim 3, **characterized in that** the rod (2a) comprises a narrowing (5a) against which the measurement unit (4a) comes to stop.

8. Measurement system according to claim 7, **characterized in that** the rod (2a) comprises an anchoring system (5b) that retains the measurement unit (4a) in the housing (3a).

9. Measurement system according to claim 1, **characterized in that** the fastening means between the measurement unit (5) and the rod (6) comprise contact surfaces between the measurement unit and the rod.

10. Measurement system according to claim 9, **characterized in that** the fastening means between the measurement unit (5) and the rod (6) comprise an adhesive substance.

11. Measurement system according to any of the previous claims, **characterized in that** the receiver means are positioned at the proximal end of the probe.

12. Measurement system according to any of claims 1 to 10, **characterized in that** the receiver means are means that may be placed on the patient's body.

13. Measurement system according to any of claims 1 to 12, **characterized in that** the remote transmission means (8, 9) and reception means are radio-frequency communication means.

14. Measurement system according to any of claims 1 to 12, **characterized in that** the remote transmission means and reception means are infrared communication means.

15. Measurement system according to any of claims 1 to 12, **characterized in that** the remote transmission means and reception means are ultrasound communication means.

16. Measurement system according to any of the above claims, **characterized in that** the measurement unit comprises a connection plane provided with conductor paths (51) that ensure electrical connection between the various components (52, 53, 54, 55) of the measurement unit.

17. Measurement system according to claim 16, **characterized in that** the connection plane consists of a flexible substrate (50) that is coiled into a tube shape and embedded in a moulding substance.

18. Measurement system according to claim 17, **characterized in that** the components (52, 53, 54, 55) of the measurement unit have electrical contacts welded onto certain conductor paths (51) selected from among said conductor paths.

19. Measurement system according to either of claims 16 or 17, **characterized in that** the components (32, 33, 34) of the measurement unit are components inserted into the measurement unit, said insertion creating electrical contact between these components and the conductor paths selected from among said conductor paths.

20. Measurement system according to claim 17, **characterized in that** as the remote transmission and reception means are radio-frequency communication means, the measurement unit comprises an antenna produced by a metallization (56) being deposited on the flexible support (50).

21. Measurement system according to any of claims 16 to 18, **characterized in that** as the remote transmission and reception means are radio-frequency communication means, the measurement unit comprises an antenna that is an added part connected to the connection plane and embedded in the moulding substance.

22. Measurement system according to any of claims 16 to 19, **characterized in that** as the remote transmission and reception means are radio-frequency communication means, the measurement unit (60, 70, 80) comprises an antenna (61, 71, 85) wound around the measurement unit.

23. Measurement system according to any of the previous claims, **characterized in that** said probe is an intubation probe capable of delivering a gas for mechanical ventilation purposes.

24. Measurement system according to any of claims 1 to 22, **characterized in that** said probe is a urinary probe capable of taking urodynamic measurements.

25. Measurement system according to any of claims 1 to 22, **characterized in that** said probe is a catheter capable of measuring intravascular pressure.

## Patentansprüche

1. System zur Messung wenigstens eines physikalischen Parameters an einer mittels einer medizinischen Sonde zugänglichen Körperstelle eines Patienten, eine medizinische Sonde (1, 6) umfassend, ausgestattet mit einem Sensor des genannten Parameters und Einrichtungen zur Lieferung eines für diesen durch den Sensor erfassten Parameter repräsentativen elektrischen Signals an eine Datenverarbeitungsvorrichtung außerhalb des Körpers des Patienten, wobei bei diesem System:
- die genannte Sonde (1, 6) durch eine Stange (2) mit Befestigungseinrichtungen gebildet wird, mit denen ein elektronischer Messmodul (4, 5, 30, 60, 70, 80) an der Stange befestigt wird,
- der Sensor (11, 12) des genannten Parameters in dem elektronischen Messmodul (4, 5, 30, 60, 70, 80) enthalten ist, der auch andere Elemente enthält, gebildet durch elektronische Einrichtungen (21, 22, 7), dem Sensor (11, 12) zugeordnet um ein Messsignal zu liefern, Femübertragungseinrichtungen (8, 9) des Messsignals, elektrische Versorgungseinrichtungen der dem Sensor zugeordneten elektronischen Einrichtungen und Fernübertragungseinrichtungen,
**dadurch gekennzeichnet,**
- **dass** der Messmodul außerdem weitere Befestigungseinrichtungen umfasst, zusätzlich zu denen der Sondenstange (2),
- **dass** die Einrichtungen zum Liefern eines repräsentativen elektrischen Signals des genannten Parameters an die Datenverarbeitungsvorrichtung Empfangseinrichtungen sind, so angeordnet, dass sie das durch die Fernübertragungseinrichtungen (8, 9) übertragene Signal empfangen können,
- **dass** die Fernübertragungseinrichtungen eine Antenne (9) in Form einer Wicklung umfassen,
- **dass** die elektrischen Versorgungseinrichtungen einen Strom- bzw. Schaltkreis umfassen, der dank der genannten Wicklung durch Fernversorgung geladen wird,
- **dass** der Sensor sich im distalen Teil der medizinischen Sonde befindet.

2. Messsystem nach Anspruch 1, **dadurch gekennzeichnet, dass** die Stange (2, 2a) einen Sitz (3, 3a) umfasst, der das Einsetzen des Messmoduls (41, 42, 4a) ermöglicht.

3. Messsystem nach Anspruch 2, **dadurch gekennzeichnet, dass** die Stange (2, 2a) hohl ist und der genannte Sitz (3, 3a) in der Innenwand der Stange vorgesehen ist.

4. Messsystem nach Anspruch 3, **dadurch gekennzeichnet, dass** der Messmodul (41, 42) ringförmig ist und der genannte Sitz (3) in der Stange (2) eine Ringnut bildet.

5. Messsystem nach Anspruch 4, **dadurch gekennzeichnet, dass** der Messmodul (41) ein geschlossener Ring ist und die Stange (2) aus einem ausreichend elastischen Material ist, so dass der Messmodul (41) durch Verformung der Stange (2) in seinen Sitz (3) eingesetzt werden kann.

6. Messsystem nach Anspruch 4, **dadurch gekennzeichnet, dass** der Messmodul (42) ein offener Ring ist, der sich nach Art eines Clips in den genannten Sitz (3) einsetzen lässt.

7. Messsystem nach Anspruch 3, **dadurch gekennzeichnet, dass** die Stange (2a) eine Verengung (5a) umfasst, die dem Messmodul (4a) als Anschlag dient.

8. Messsystem nach Anspruch 7, **dadurch gekennzeichnet, dass** die Stange (2a) ein Verankerungssystem (5b) umfasst, das den Messmodul (4a) in seinem Sitz (3a) festhält.

9. Messsystem nach Anspruch 1, **dadurch gekennzeichnet, dass** die Befestigungseinrichtungen zwischen dem Messmodul (5) und der Stange (6) Kontaktaufnahmeflächen zwischen dem Messmodul und der Stange umfassen.

10. Messsystem nach Anspruch 9, **dadurch gekennzeichnet, dass** die Befestigungseinrichtungen zwischen dem Messmodul (5) und der Stange (6) eine haftende Substanz umfassen.

11. Messsystem nach einem der vorangehenden Ansprüche, **dadurch gekennzeichnet, dass** die Empfangseinrichtungen in dem proximalen Ende der Sonde angeordnet sind.

12. Messsystem nach einem der Ansprüche 1 bis 10, **dadurch gekennzeichnet, dass** die Empfangseinrichtungen Einrichtungen sind, die am bzw. im Körper des Patienten angebracht werden können.

13. Messsystem nach einem der Ansprüche 1 bis 12, **dadurch gekennzeichnet, dass** die Fernübertragungseinrichtungen (8, 9) und die Empfangseinrichtungen Funkfrequenz-Kommunikationseinrichtungen sind.

14. Messsystem nach einem der Ansprüche 1 bis 12, **dadurch gekennzeichnet, dass** die Femübertragungseinrichtungen und die Empfangseinrichtungen Infrarot-Kommunikationseinrichtungen sind.

15. Messsystem nach einem der Ansprüche 1 bis 12, **dadurch gekennzeichnet, dass** die Fernübertragungseinrichtungen und die Empfangseinrichtungen Ultraschall-Kommunikationseinrichtungen sind.

16. Messsystem nach einem der vorangehenden Ansprüche, **dadurch gekennzeichnet, dass** der Messmodul eine mit Leiterbahnen (51) versehene Verbindungsebene umfasst, die ermöglichen, elektrische Verbindungen zwischen den verschiedenen Elementen (52, 53, 54, 55) des Messmoduts herzustellen.

17. Messsystem nach Anspruch 16, **dadurch gekennzeichnet, dass** die Verbindungsebene durch einen flexiblen Träger (50) gebildet wird, röhrenförmig gerollt und eingebettet in eine Gießsubstanz.

18. Messsystem nach Anspruch 17, **dadurch gekennzeichnet, dass** die Elemente (52, 53, 54, 55) des Messmoduls elektrische Kontakte haben, die auf entsprechende unter den Leiterbahnen enthaltene Leitbahnen (51) gelötet sind.

19. Messsystem nach einem der Ansprüche 16 oder 17, **dadurch gekennzeichnet, dass** die Elemente (32, 33, 34) des Messmoduls Elemente sind, die in dem Messmodul durch Einführung angebracht werden, wobei die Einführung elektrische Kontakte zwischen diesen Elementen und entsprechenden Leiterbahnen unter den genannten Leiterbahnen herstellt.

20. Messsystem nach Anspruch 17, **dadurch gekennzeichnet, dass** die Fernübertragungseinrichtungen und die Empfangseinrichtungen Funkfrequenz-Kommunikationseinrichtungen sind, wobei der Messmodul eine durch eine Metallisierung (56) realisierte Antenne umfasst, abgeschieden auf dem flexiblen Träger (50).

21. Messsystem nach einem der Ansprüche 16 bis 18, **dadurch gekennzeichnet, dass** die Fernübertragungseinrichtungen und die Empfangseinrichtungen Funkfrequenz-Kommunikationseinrichtungen sind und der Messmodul die genannte Antenne umfasst, die ein angefügtes, mit der Verbindungsebene verbundenes und in die Gießsubstanz eingebettetes Element ist.

22. Messsystem nach einem der Ansprüche 16 bis 19, **dadurch gekennzeichnet, dass** die Fernübertragungseinrichtungen und die Empfangseinrichtungen Funkfrequenz-Kommunikationseinrichtungen sind und der genannte Messmodul (60, 70, 80) die um den Messmodul herum angebrachte Antenne (61, 71, 85) umfasst.

23. Messsystem nach einem der vorangehenden Ansprüche, **dadurch gekennzeichnet, dass** die genannte Sonde eine Intubationssonde ist, fähig im Falle einer künstlichen Ventilation ein Gas zu liefern.

24. Messsystem nach einem der Ansprüche 1 bis 22, **dadurch gekennzeichnet, dass** die genannte Sonde eine für urodynamische Messungen dienende Harnleitersonde ist.

25. Messsystem nach einem der Ansprüche 1 bis 22, **dadurch gekennzeichnet, dass** die genannte Sonde ein Katheder ist, fähig den Arteriendruck zu messen.
